# EUROPEAN PATENT SPECIFICATION

(11) **EP 1 186 672 B1**
(45) Date of publication and mention of the grant of the patent: **30.11.2005**
(21) Application number: 01306983.6
(22) Date of filing: 17.08.2001
(51) Int. Cl.: C12Q 1/68, C07K 14/47, C07K 16/18

(54) **Polymorphisms in the human organic anion transporter C (OATP-C) gene**
Polymorphismen im Gen für den humanen organischen Anionentransporter C (OATP-C)
Polymorphismes dans le gène humain du transporteur d'anion organique (OATP-C)

(30) Priority: 23.08.2000 US 226909 P
(43) Date of publication of application: 13.03.2002
(60) Divisional application: 05011183.0
(73) Proprietor: AstraZeneca AB, 151 85 Södertälje (SE)
(72) Inventor: Adeokun, Monisola, Cheshire, SK10 4TG (GB); Ambrose, Helen Jean, Cheshire, SK10 4TG (GB); Cresswell, Carl John, Cheshire, SK10 4TG (GB); Dudley, Adam Jeston, Wilmington, DE 19850-5437 (US)
(74) Representative: Giles, Allen Frank

(56) References cited:
- WO-A-00/08157
- WO-A-99/06046
- TAMAI I ET AL: "MOLECULAR IDENTIFICATION AND CHARACTERIZATION OF NOVEL MEMBERS OF THE HUMAN ORGANIC ANION TRANSPORTER (OATP) FAMILY" BIOCHIMICA ET BIOPHYSICA ACTA, AMSTERDAM, NL, vol. 273, no. 1, 2000, pages 251-260, XP000941538 ISSN: 0006-3002 & DATABASE EMBL [Online] EBI; 8 June 1999 (1999-06-08) TAMAI I ET AL.: "Homo sapiens mRNA for organic anion transporter OATP-C" retrieved from HTTP://WWW.EBI.AC.UK/CGI-BIN/EMBLFETCH Database accession no. AB026257
- DATABASE EMBL [Online] EBI; 2 February 2000 (2000-02-02) MUZNY DM ET AL.: "Homo sapiens 12p BAC RP11-12505" retrieved from HTTP://WWW.EBI.AC.UK/CGI-BIN/EMBLFETCH Database accession no. AC022335 XP002188259
- HSIANG B ET AL: "A NOVEL HUMAN HEPATIC ORGANIC ANION TRANSPORTING POLYPEPTIDE (OATP2)" JOURNAL OF BIOLOGICAL CHEMISTRY, AMERICAN SOCIETY OF BIOLOGICAL CHEMISTS, BALTIMORE, MD, US, vol. 274, no. 52, 1999, pages 37161-37168, XP000877290 ISSN: 0021-9258
- ULBRECHT M ET AL.: "Assoziation of beta 2-adrenoreceptor variants with bronchial hyperresponsiveness" AMERICAN JOURNAL OF RESPIRATORY AND CRITICAL CARE MEDICINE, vol. 161, no. 2, February 2000 (2000-02), pages 469-474, XP002188594

### SEQUENCE LISTING

<110> AstraZeneca AB
<120> Chemical Compounds
<130> adeokun
   ambrose
   cresswell
   dudley
<140>
   <141>
<160> 12
<170> PatentIn Ver. 2.1
<210> 1
   <211> 2452
   <212> DNA
   <213> Homo sapiens
<400> 1
   gtggacttgt tgcagttgct gtaggattct aaatccaggt gattgtttca aactgagcat 60
   caacaacaaa aacatttgta tgatatctat atttcaatca tggaccaaaa tcaacatttg 120
   aataaaacag cagaggcaca accttcagag aataagaaaa caagatactg caatggattg 180
   aagatgttct tggcagctct gtcactcagc tttattgcta agacactagg tgcaattatt 240
   atgaaaagtt ccatcattca tatagaacgg agatttgaga tatcctcttc tcttgttggt 300
   tttattgacg gaagctttga aattggaaat ttgcttgtga ttgtatttgt gagttacttt 360
   ggatccaaac tacatagacc aaagttaatt ggaatcggtt gtttcattat gggaattgga 420
   ggtgttttga ctgctttgcc acatttcttc atgggatatt acaggtattc taaagaaact 480
   aatatcaatt catcagaaaa ttcaacatcg accttatcca cttgtttaat taatcaaatt 540
   ttatcactca atagagcatc acctgagata gtgggaaaag gttgtttaaa ggaatctggg 600
   tcatacatgt ggatatatgt gttcatgggt aatatgcttc gtggaatagg ggagactccc 660
   atagtaccac tggggctttc ttacattgat gatttcgcta aagaaggaca ttcttctttg 720
   tatttaggta tattgaatgc aatagcaatg attggtccaa tcattggctt taccctggga 780
   tctctgtttt ctaaaatgta cgtggatatt ggatatgtag atctaagcac tatcaggata 840
   actcctactg attctcgatg ggttggagct tggtggctta atttccttgt gtctggacta 900
   ttctccatta tttcttccat accattcttt ttcttgcccc aaactccaaa taaaccacaa 960
   aaagaaagaa aagcttcact gtctttgcat gtgctggaaa caaatgatga aaaggatcaa 1020
   acagctaatt tgaccaatca aggaaaaaat attaccaaaa atgtgactgg ttttttccag 1080
   tcttttaaaa gcatccttac taatcccctg tatgttatgt ttgtgctttt gacgttgtta 1140
   caagtaagca gctatattgg tgcttttact tatgtcttca aatacgtaga gcaacagtat 1200
   ggtcagcctt catctaaggc taacatctta ttgggagtca taaccatacc tatttttgca 1260
   agtggaatgt ttttaggagg atatatcatt aaaaaattca aactgaacac cgttggaatt 1320
   gccaaattct catgttttac tgctgtgatg tcattgtcct tttacctatt atattttttc 1380
   atactctgtg aaaacaaatc agttgccgga ctaaccatga cctatgatgg aaataatcca 1440
   gtgacatctc atagagatgt accactttct tattgcaact cagactgcaa ttgtgatgaa 1500
   agtcaatggg aaccagtctg tggaaacaat ggaataactt acatctcacc ctgtctagca 1560
   ggttgcaaat cttcaagtgg caataaaaag cctatagtgt tttacaactg cagttgtttg 1620
   gaagtaactg gtctccagaa cagaaattac tcagcccatt tgggtgaatg cccaagagat 1680
   gatgcttgta caaggaaatt ttactttttt gttgcaatac aagtcttgaa tttatttttc 1740
   tctgcacttg gaggcacctc acatgtcatg ctgattgtta aaattgttca acctgaattg 1800
   aaatcacttg cactgggttt ccactcaatg gttatacgag cactaggagg aattctagct 1860
   ccaatatatt ttggggctct gattgataca acgtgtataa agtggtccac caacaactgt 1920
   ggcacacgtg ggtcatgtag gacatataat tccacatcat tttcaagggt ctacttgggc 1980
   ttgtcttcaa tgttaagagt ctcatcactt gttttatata ttatattaat ttatgccatg 2040
   aagaaaaaat atcaagagaa agatatcaat gcatcagaaa atggaagtgt catggatgaa 2100
   gcaaacttag aatccttaaa taaaaataaa cattttgtcc cttctgctgg ggcagatagt 2160
   gaaacacatt gttaagggga gaaaaaaagc cacttctgct tctgtgtttc caaacagcat 2220
   tgcattgatt cagtaagatg ttatttttga ggagttcctg gtcctttcac taagaatttc 2280
   cacatctttt atggtggaag tataaataag cctatgaact tataataaaa caaactgtag 2340
   gtagaaaaaa tgagagtact cattgtacat tatagctaca tatttgtggt taaggttaga 2400
   ctatatgatc catacaaatt aaagtgagag acatggttac tgtgtaataa aa         2452
<210> 2
   <211> 691
   <212> PRT
   <213> Homo sapiens
<400> 2
   Met Asp Gln Asn Gln His Leu Asn Lys Thr Ala Glu Ala Gln Pro Ser
    1               5                  10                  15
   Glu Asn Lys Lys Thr Arg Tyr Cys Asn Gly Leu Lys Met Phe Leu Ala
               20                  25                  30
   Ala Leu Ser Leu Ser Phe Ile Ala Lys Thr Leu Gly Ala Ile Ile Met
           35                  40                  45
   Lys Ser Ser Ile Ile His Ile Glu Arg Arg Phe Glu Ile Ser Ser Ser
       50                  55                  60
   Leu Val Gly Phe Ile Asp Gly Ser Phe Glu Ile Gly Asn Leu Leu Val
   65                  70                  75                  80
   Ile Val Phe Val Ser Tyr Phe Gly Ser Lys Leu His Arg Pro Lys Leu
                   85                  90                  95
   Ile Gly Ile Gly Cys Phe Ile Met Gly Ile Gly Gly Val Leu Thr Ala
               100                 105                 110
   Leu Pro His Phe Phe Met Gly Tyr Tyr Arg Tyr Ser Lys Glu Thr Asn
           115                 120                 125
   Ile Asn Ser Ser Glu Asn Ser Thr Ser Thr Leu Ser Thr Cys Leu Ile
       130                 135                 140
   Asn Gln Ile Leu Ser Leu Asn Arg Ala Ser Pro Glu Ile Val Gly Lys
   145                 150                 155                 160
   Gly Cys Leu Lys Glu Ser Gly Ser Tyr Met Trp Ile Tyr Val Phe Met
                   165                 170                 175
   Gly Asn Met Leu Arg Gly Ile Gly Glu Thr Pro Ile Val Pro Leu Gly
               180                 185                 190
   Leu Ser Tyr Ile Asp Asp Phe Ala Lys Glu Gly His Ser Ser Leu Tyr
           195                 200                 205
   Leu Gly Ile Leu Asn Ala Ile Ala Met Ile Gly Pro Ile Ile Gly Phe
       210                 215                 220
   Thr Leu Gly Ser Leu Phe Ser Lys Met Tyr Val Asp Ile Gly Tyr Val
   225                 230                 235                 240
   Asp Leu Ser Thr Ile Arg Ile Thr Pro Thr Asp Ser Arg Trp Val Gly
                   245                 250                 255
   Ala Trp Trp Leu Asn Phe Leu Val Ser Gly Leu Phe Ser Ile Ile Ser
               260                 265                 270
   Ser Ile Pro Phe Phe Phe Leu Pro Gln Thr Pro Asn Lys Pro Gln Lys
           275                 280                 285
   Glu Arg Lys Ala Ser Leu Ser Leu His Val Leu Glu Thr Asn Asp Glu
       290                 295                 300
   Lys Asp Gln Thr Ala Asn Leu Thr Asn Gln Gly Lys Asn Ile Thr Lys
   305                 310                 315                 320
   Asn Val Thr Gly Phe Phe Gln Ser Phe Lys Ser Ile Leu Thr Asn Pro
                   325                 330                 335
   Leu Tyr Val Met Phe Val Leu Leu Thr Leu Leu Gln Val Ser Ser Tyr
               340                 345                 350
   Ile Gly Ala Phe Thr Tyr Val Phe Lys Tyr Val Glu Gln Gln Tyr Gly
           355                 360                 365
   Gln Pro Ser Ser Lys Ala Asn Ile Leu Leu Gly Val Ile Thr Ile Pro
       370                 375                 380
   Ile Phe Ala Ser Gly Met Phe Leu Gly Gly Tyr Ile Ile Lys Lys Phe
   385                 390                 395                 400
   Lys Leu Asn Thr Val Gly Ile Ala Lys Phe Ser Cys Phe Thr Ala Val
                   405                 410                 415
   Met Ser Leu Ser Phe Tyr Leu Leu Tyr Phe Phe Ile Leu Cys Glu Asn
               420                 425                 430
   Lys Ser Val Ala Gly Leu Thr Met Thr Tyr Asp Gly Asn Asn Pro Val
           435                 440                 445
   Thr Ser His Arg Asp Val Pro Leu Ser Tyr Cys Asn Ser Asp Cys Asn
       450                 455                 460
   Cys Asp Glu Ser Gln Trp Glu Pro Val Cys Gly Asn Asn Gly Ile Thr
   465                 470                 475                 480
   Tyr Ile Ser Pro Cys Leu Ala Gly Cys Lys Ser Ser Ser Gly Asn Lys
                   485                 490                 495
   Lys Pro Ile Val Phe Tyr Asn Cys Ser Cys Leu Glu Val Thr Gly Leu
               500                 505                 510
   Gln Asn Arg Asn Tyr Ser Ala His Leu Gly Glu Cys Pro Arg Asp Asp
           515                 520                 525
   Ala Cys Thr Arg Lys Phe Tyr Phe Phe Val Ala Ile Gln Val Leu Asn
       530                 535                 540
   Leu Phe Phe Ser Ala Leu Gly Gly Thr Ser His Val Met Leu Ile Val
   545                 550                 555                 560
   Lys Ile Val Gln Pro Glu Leu Lys Ser Leu Ala Leu Gly Phe His Ser
                   565                 570                 575
   Met Val Ile Arg Ala Leu Gly Gly Ile Leu Ala Pro Ile Tyr Phe Gly
               580                 585                 590
   Ala Leu Ile Asp Thr Thr Cys Ile Lys Trp Ser Thr Asn Asn Cys Gly
           595                 600                 605
   Thr Arg Gly Ser Cys Arg Thr Tyr Asn Ser Thr Ser Phe Ser Arg Val
       610                 615                 620
   Tyr Leu Gly Leu Ser Ser Met Leu Arg Val Ser Ser Leu Val Leu Tyr
   625                 630                 635                 640
   Ile Ile Leu Ile Tyr Ala Met Lys Lys Lys Tyr Gln Glu Lys Asp Ile
                   645                 650                 655
   Asn Ala Ser Glu Asn Gly Ser Val Met Asp Glu Ala Asn Leu Glu Ser
               660                 665                 670
   Leu Asn Lys Asn Lys His Phe Val Pro Ser Ala Gly Ala Asp Ser Glu
           675                 680                 685
   Thr His Cys
   690
<210> 3
   <211> 1538
   <212> DNA
   <213> Homo sapiens
<400> 3
   atgctctttg acctctgaaa atattggaga attttacaac tggcaccttt agctcaggat 60
   tataaaggtt gttagttagt ttgtactgtt ttatcttcat tgtatataat atatatatta 120
   gtctccaaac atgttgatgt gttttcaatg aaatggatgt ctgaggagaa aaccattagc 180
   ctgagaaaac ccaaactgta ttcccattgt gaataaaagg aagtccataa aaatgatgga 240
   aaatgttctg cattcctgtt atgatatcaa aatctggcag tacatgaaaa tttttcaaag 300
   tgcttattta acaggcataa tctttggtct cctgagccag aatctgctgg gtatgggact 360
   ggattgctat tttgacaact cgccagtaga ttcttactca gcagagtatt tggaagcctt 420
   actctaatat tttggccttg ggtctacatt tctcagttct gcacagtcat tcttcccctc 480
   tacactactc tttagtttgt ctcatgattc caatactctc aataattaac caagaataga 540
   actaatcaat cagataactg tggcacagac atcaaataca ttttgctgca accatatcaa 600
   caaatgtccc atgaatgata aggggtaacc atattctcat atatgcatcc tcacattacc 660
   acatatatat atgtgcatat gtgtatacag gtaaaagtgt gtatatatgt atacatgtat 720
   gtttgtgtgt atatacatac atatatcttc acacttttct gaaatatata tatttatgtg 780
   agagaagggt ctgtacttta tttcagaaga gagcttaatg tccaaggtat aattgagagt 840
   ctaaaatgtt tgagttattg aattaattaa acttcatctc tactcaagaa aacttttaac 900
   tgagttaagc tcttcctttc tccacaagtc aagtcaataa aaggaaactg tgatattaat 960
   aattctttcc tgttttgatg taaagaatct atcgcataaa gcagtcttaa ttttcatcat 1020
   tcagaaaaat ggtcttgcag ttaattggga ctctcttatt ccaggtggta tctccagtct 1080
   ccatacatac cacgttagaa ccatacttat gtaccaagca aagagggtat attttaattt 1140
   ttaaatgcca atgtaacctg taggcatatt ttttatttgt cttaaattat ttcctatttg 1200
   gaagttttaa atacctggaa taatttattg tactcatatt tttaaagaaa aaaatcttat 1260
   gccaccaact taattgaata aacaagtaaa agccattccc aaaagtaagg tttacttgtt 1320
   aagattaaca aaaaataatg tgagaattct gagaaatata atctttaaat attggcaact 1380
   ggagtgaact cttaaaacta actaggtttt atatgtttga ctagagcaat gacataataa 1440
   ggtggttaat catcactgga cttgttttca aaaagccaac tactttaaga ggaataaagg 1500
   gtggacttgt tgcagttgct gtaggattct aaatccag                         1538
<210> 4
   <211> 200
   <212> DNA
   <213> Homo sapiens
<400> 4
   gatactgcaa tggattgaag gtagaataag ttttatgttt ttgagctaaa ataagtaaat 60
   agggaacttt aatgtataga aaagcaagtt gttaaaaaga acattatgtt tcaaattata 120
   attttcaatt gaagcatata ttgaaatatt aacataatga ttcatacctt gatttaaacc 180
   agtcttttaa tctgattaag                                             200
<210> 5
   <211> 300
   <212> DNA
   <213> Homo sapiens
<400> 5
   tgacggaagc tttgaaattg gtaacattta ttttctattt taataaccaa acttgcaaag 60
   ttaaaaaata tatatgcttt acaccactgg ttatcaactg gggtaaattt atctctcaca 120
   ggcaatttgg caataactaa aaacatttgt ggttgtcata actgcacagg ggttgggggc 180
   aatggaagtg ctactggtat ctaaaggtag aggtcagggg tactgctaaa tattctataa 240
   tgcacaaaga atgatgtaac tgaaaatgtt gatagtgagg atgttcagaa accctgattc 300
<210> 6
   <211> 300
   <212> DNA
   <213> Homo sapiens
<400> 6
   ccacatttct tcatgggata gtaagtgtta aaaaaaaaaa aaacctctgt gccactatca 60
   gtaccttgta aattaggagt agaattttat tattatccct ttaaataggc agttaccttt 120
   tgagaagata cccactaagt gtgtacagaa atgaaatagt gtctatttgt ctacataatc 180
   attttattta tcgtagcttt catatacttt gaaataacaa aaagactaaa ctgtagagtt 240
   tcaaatgaaa taaataggct ttttatgaat ttttagtata acgtatatac tgtacgtctt 300
<210> 7
   <211> 300
   <212> DNA
   <213> Homo sapiens
<400> 7
   acctgagata gtgggaaaag gtaagaatta atattgacag taaaaagtct tctaaaatgt 60
   atacatttaa ttacatctct aaaaattgtt gtgatattca ttagcaaaat ttaattaaga 120
   atgaatagga aaaacatttg actcttacag acataattat agtgttaata tacacagttc 180
   gcccattaac aacacaggtt taaactacgc gttttcactt ctatgcaaat tttgtccatc 240
   tgaactggat gataaacctg ccggtaagaa tatctgacat tttctatatt tggattgaac 300
<210> 8
   <211> 200
   <212> DNA
   <213> Homo sapiens
<400> 8
   tagcagcata agaatggact aatacaccat attgtcaaag tttgcaaagt gaatataaat 60
   tacttgtact tgtaaattaa aaaaaaataa gtagaataat taagagttta caagtagtta 120
   aatttgtaat agaaatgcta aaattaatgt ttaaaatgaa acactctctt atctacatag 180
   gttgtttaaa ggaatctggg 200
<210> 9
   <211> 200
   <212> DNA
   <213> Homo sapiens
<400> 9
   tattggatat gtagatctaa gtaagtacaa ccagaacaag gtaccatgat aacgtctttc 60
   taagcacaca tgcgaaaaac attttttcaa ataactgaat tcactctttc aatagtcctt 120
   tgcttaatat aattagaaag ttacaagtag gaaataaatg tattactaat cagaataaat 180
   ataaaatcca gctcctattt 200
<210> 10
   <211> 203
   <212> DNA
   <213> Homo sapiens
<400> 10
   ttaaaaaaaa ctttgccatt tcgtcatcat caaagcaaat ttcttcatat aaagaaaaat 60
   tctttatcta ctttttcttt tccctctttc tctgctttca ctttacttct tccttctcct 120
   ccccttcttt gtctttttct tctctctctc tctttttgat atatgtctat catatatttc 180
   cagaaataat ccagtgacat ctc                                         203
<210> 11
   <211> 201
   <212> DNA
   <213> Homo sapiens
<400> 11
   catgtcatgc tgattgttaa gtaagtatga cttttaaaaa cattttcata tgcatgagac 60
   tataaacaca cctaatgata tgcatatttt tacataatat actgggaatt caaattcata 120
   tttcatcaaa ttttaatttt ctgagaattc attttattaa aatttactat gaactctcaa 180
   ggctgtaatt aataattttg c                                           201
<210> 12
   <211> 200
   <212> DNA
   <213> Homo sapiens
<400> 12
   tgatttgggt ctttgagatt tctaataatc tttattattg ggtagatgca gaacaaaata 60
   ataaacgaat cctccaaatt tttgaacttt tatttaatca aaatatatca atgtggaata 120
   tcatgcagtt acatttaaaa tatgttccct aaactgacat cttctcttct cctattacag 180
   gaggaattct agctccaata                                             200

## Description

This invention relates to polymorphisms in the human OATPC gene and corresponding novel allelic polypeptides encoded thereby. The invention also relates to methods and materials for analysing allelic variation in the OATPC gene, and to the use of OATPC polymorphism in treatment of diseases with OATPC transportable drugs.

Na+-independent organic anion transporting polypeptide (OATP) C gene is a member of the OATP supergene family involved in multifunctional transport of organic anion. OATPC tranports the organic anion taurocholate, conjugated steroids: DHEAS, estradiol 17β-D-glucoronide and estrone-3-sulfate, eicosanoids: PGE₂, thromboxane B₂, leukotriene C₄, and E₄, and thyroid hormones T4 and T3 ^{1,2}. OATPC has also been shown to be involved in the transport of xenobiotics , and drugs involved in lipid lowering *e.g.* statins¹. Statins have been refered to as a first-line therapy for patients with atherosclerotic vascular diseases. The OATPC gene and its product is also thought to be of importance in other diseases due to its transport of DHEAS an adrenal steroid which has been suggested to have positive neuropsychiatric, immune, and metabolic effects³. Due to the substrate specificity, location in the liver, and being exclusively expressed in the liver, Abe *et al* suggested that OATPC could be the predominant clearance mechanism of several endogenous and exogenous substrates in the liver. OATPC is the first human molecule reported to transport thyroid hormones².
² Identification of a Novel Gene Family Encoding Human liver-specifc Organic Anion Transporter LST-1, Takaaki Abe *et al* J Biol Chem **274,** 17159-17163 (1999)
¹A Novel Human Hepatic Organic Anion Transporting Polypeptide (OATP2), Hsiang *et al* J Biol Chem **274,** 37161-37168 (1999)
³ Bates *et al* (1998) Curr. Opin. Endocrinol. Diab. **5,** 357-366

This liver specific transporter may be useful in liver-specific drug delivery systems and liver-specific chemotherapy, bile acid formation and the pathogenesis of diseases such as cholestasis, hyperbilirubinemia and thyroid hormone resistance.

The OATPC gene (sometimes called OAPT2 in the literature) has been cloned by four different groups, annotated and published as EMBL accession numbers AB026257 (OATPC, 2452bp), AF205071(OATP2, 2830, ref 1), AJ132573(OATP2, 2778)⁴ and AF060500 (LST-1)². Polymorphism has been reported by Tamai⁵ which is Asn 130Asp and Val174Ala although any functional effect was stated therein to be not clear. Konig (2000) J Biol Chem 275, 23161-23168 describes the genomic organisation of OATP 1, 2 and 8. International patent application WO 00/08157 describes human anion transporter genes and some polymorphisms.
⁴ A novel human organic anion transporting polypeptide localised to the basolateral hepatocyte membrane, Konig Jorg *et al* (2000) Am J Physiol. Gastrointest. Liver Physiol. **278:** G156-G164
⁵Tamai *et al* (2000), BBRC, 273, 251-60

All positions herein of polymorphisms in the OATPC polynucleotide relate to the position in one of SEQ ID NO 1 or 3-12 unless stated otherwise or apparent from the context.

All positions herein of polymorphisms in the OATPC polypeptide relate to the position in SEQ ID NO 2 unless stated otherwise or apparent from the context.

One approach is to use knowledge of polymorphisms to help identify patients most suited to therapy with particular pharmaceutical agents (this is often termed "pharmacogenetics"). Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al.* (1997), Clinical Chemistry, **43,** 254; Marshall (1997), Nature Biotechnology, **15,** 1249; International Patent Application WO 97/40462, Spectra Biomedical; and Schafer *et al*. (1998), Nature Biotechnology, **16,** 33.

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature), position, new amino acid. For (a hypothetical) example "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of polymorphisms in OATPC.

According to one aspect of the present invention there is provided a method for the detection of a polymorphism in OATPC in a human, which method comprises determining the sequence of the human at:
position 1561 of the OATPC gene as defined by the position in SEQ ID NO: 1 is not G; or
position 488 in OATPC polypeptide defined by position in SEQ ID NO: 2 is not Gly.

The term human includes both a human having or suspected of having a OATPC mediated disease and an asymptomatic human who may be tested for predisposition or susceptibility to such disease. At each position the human may be homozygous for an allele or the human may be a heterozygote.

The term polymorphism includes single nucleotide substitution, nucleotide insertion and nucleotide deletion which in the case of insertion and deletion includes insertion or deletion of one or more nucleotides at a position of a gene.

The method for diagnosis is preferably one in which the sequence is determined by a method selected from amplification refractory mutation system and restriction fragment length polymorphism.

The status of the individual may be determined by reference to allelic variation at any one, two, three, four, five, six, seven, eight, nine or more positions.

The test sample of nucleic acid is conveniently a sample of blood, bronchoalveolar lavage fluid, sputum, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau *et al*., Clin. Chem. **43,** 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

### Abbreviations:

| | |
|---|---|
| ALEX™ | Amplification refractory mutation system linear extension |
| APEX | Arrayed primer extension |
| ARMS™ | Amplification refractory mutation system |
| b-DNA | Branched DNA |
| bp | base pair |
| CMC | Chemical mismatch cleavage |
| COPS | Competitive oligonucleotide priming system |
| DGGE | Denaturing gradient gel electrophoresis |
| FRET | Fluorescence resonance energy transfer |
| HMG-CoA | 3-hydroxy-3-methylglutaryl-coenzyme A |
| LCR | Ligase chain reaction |
| MASDA | Multiple allele specific diagnostic assay |
| NASBA | Nucleic acid sequence based amplification |
| OATP | Na+-independent organic anion transporting polypeptide |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

**General:** DNA sequencing, Sequencing by hybridisation
**Scanning:** PTT*, SSCP, DGGE, TGGE, Cleavase, Heteroduplex analysis, CMC, Enzymatic mismatch cleavage
* Note: not useful for detection of promoter polymorphisms.

### Hybridisation Based

Solid phase hybridisation: Dot blots, MASDA, Reverse dot blots,
Oligonucleotide arrays (DNA Chips)
Solution phase hybridisation: Taqman™ - US-5210015 & US-5487972 (Hoffmann-La Roche), Molecular Beacons - Tyagi *et al* (1996), Nature Biotechnology, 14, 303; WO 95/13399 (Public Health Inst., New York)
**Extension Based:** ARMS™, ALEX™ - European Patent No. EP 332435 B1 (Zeneca Limited), COPS - Gibbs *et al* (1989), Nucleic Acids Research, 17, 2347.
**Incorporation Based:** Mini-sequencing, APEX
**Restriction Enzyme Based: RFLP, Restriction site generating PCR**
**Ligation Based: OLA**
**Other: Invader assay**

### Table 2 - Signal Generation or Detection Systems

**Fluorescence:** FRET, Fluorescence quenching, Fluorescence polarisation - United Kingdom
Patent No. 2228998 (Zeneca Limited)
**Other:** Chemiluminescence, Electrochemiluminescence, Raman, Radioactivity, Colorimetric, Hybridisation protection assay, Mass spectrometry

### Table 3 - Further Amplification Methods

SSR, NASBA, LCR, SDA, b-DNA

Preferred mutation detection techniques include ARMS™, ALEX™, COPS, Taqman, Molecular Beacons, RFLP, and restriction site based PCR and FRET techniques.

Particularly preferred methods include ARMS™ and RFLP based methods. ARMS™ is an especially preferred method.

In a further aspect, the diagnostic methods of the invention are used to assess the pharmacogenetics of a drug transportable by OATPC.

Assays, for example reporter-based assays, may be devised to detect whether one or more of the above polymorphisms affect transcription levels and/or message stability.

Individuals who carry particular allelic variants of the OATPC gene may therefore exhibit differences in their ability to regulate protein biosynthesis under different physiological conditions and will display altered abilities to react to different diseases. In addition, differences arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by OATPC. This may be particularly relevant in the development of hyperlipoproteinemia and cardiovascular disease and the present invention may be used to recognise individuals who are particularly at risk from developing these conditions.

In a further aspect, the diagnostic methods of the invention are used in the development of new drug therapies which selectively target one or more allelic variants of the OATPC gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a human OATPC gene or its complementary strand comprising a variant allelic polymorphism at one or more of positions defined herein or a fragment thereof of at least 20 bases comprising at least one novel polymorphism.

Fragments are at least 17 bases, more preferably at least 20 bases, more preferably at least 30 bases.

According to another aspect of the present invention there is provided a polynucleotide comprising at least 20 bases of the human OATPC gene and comprising an allelic variant selected from any one of the following:

| Region | variant | Position in SEQ ID NO | SEQ ID NO |
|---|---|---|---|
| Exon 10 | C | 1561 | 1 |

According to another aspect of the present invention there is provided a human OATPC gene or its complementary strand comprising a polymorphism, preferably corresponding with one or more the positions defined herein or a fragment thereof of at least 20 bases comprising at least one polymorphism.

Fragments are at least 17 bases, more preferably at least 20 bases, more preferably at least 30 bases.

The invention further provides a nucleotide primer which can detect a polymorphism of the invention.

According to another aspect of the present invention there is provided an ARMS allele specific primer or an allele-specific oligonucleotide probe selected from one of the following:
an ARMS allele specific primer;
or an allele-specific oligonucleotide probe;
capable of detecting a OATPC gene polymorphism, preferably at one or more of the positions as defined herein.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a OATPC gene polymorphism, preferably at one or more of the positions defined herein.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided an allele specific primer or an allele specific oligonucleotide probe capable of detecting a OATPC gene polymorphism at one of the positions defined herein.

In another aspect of the invention, the single nucleotide polymorphisms of this invention may be used as genetic markers in linkage studies. This particularly applies to the polymorphisms of relatively high frequency. The OATPC gene is on chromosome 12p (as shown from a database search with the cDNA as a query sequence). Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. ***Ann Hum Genet*** (1998) **62:**481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. *Blood* (1999) **93:**4277-83, Keightley AM, Lam YM, Brady JN, Cameron CL, Lillicrap D).

According to another aspect of the present invention there is provided use of a statin in preparation of a medicament for treating a cardiovascular disease in a human detected as having an OATPC polymorphism at one or more of the following positions:
position 1561 of the OATPC gene as defined by the position in SEQ ID NO: 1 is not G;
position 488 in OATPC polypeptide defined by position in SEQ ID NO: 2 is not Gly.

Preferably determination of the status of the human is clinically useful. Examples of clinical usefulness include deciding which statin drug or drugs to administer and/or in deciding on the effective amount of the statin drug or drugs. Statins already approved for use in humans include atorvastatin, cerivastatin, fluvastatin, pravastatin and simvastatin. The reader is referred to the following references for further information: Drugs and Therapy Perspectives (12^{th} May 1997), **9:** 1-6; Chong (1997) Pharmacotherapy **17:** 1157-1177; Kellick (1997) Formulary **32:** 352; Kathawala (1991) Medicinal Research Reviews, **11**: 121-146; Jahng (1995) Drugs of the Future **20**: 387-404, and Current Opinion in Lipidology, (1997), **8**, 362 - 368. A preferred statin drug is compound 3a (S-4522) in Watanabe (1997) Bioorganic and Medicinal Chemistry **5**: 437-444; now called rosuvastatin, see Olsson (2001) American Journal of Cardiology, 87, supplement 1, 33-36. The term "drug transportable by OATPC" means that transport by OATPC in humans is an important part of a drug exerting its pharmceutical effect in man. For example, some statins have to be transported to the liver by OATPC to exert their lipid lowering effects.

According to another aspect of the present invention there is provided an allelic variant of human OATPC polypeptide comprising:
an arginine at position 488 of SEQ ID NO 2;
or a fragment thereof comprising at least 10 amino acids provided that the fragment comprises at least one allelic variant.

Fragments of polypeptide are at least 10 amino acids, more preferably at least 15 amino acids, more preferably at least 20 amino acids.

The invention will now be illustrated but not limited by reference to the following Examples. All temperatures are in degrees Celsius.

In the Examples below, unless otherwise stated, the following methodology and materials have been applied.

AMPLITAQ™ ,available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Sp ring Harbor Laboratory, 1989).

Electropherograms were obtained in a standard manner: data was collected by ABI377 data collection software and the wave form generated by ABI Prism sequencing analysis (2.1.2).

### Example 1

### Identification of Polymorphisms

### 1. Methods

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR. Where described below, the primary fragment was diluted 1/100 and two microlitres were used as template for amplification of secondary fragments. PCR was performed in two stages (primary fragment then secondary fragment) to ensure specific amplification of the desired target sequence.

| **Polymorphisms in OATPC: cDNA screening of 15 Liver samples** | | | | |
|---|---|---|---|---|
| **Region** | **SNP** | **Position** | **Amino Acid Change** | **Allele frequencies** |
| Exon 4 | G/A | 510 | None | G=96.7% A=3.3% |
| Exon 5 | C/T | 670 | None | C=50% T=50% |
| Exon 5 | C/T | 696 | None | C=60% T=40% |
| Exon 9 | C/G | 1299 | Phe400Leu | C=96.7% G=3.3% |
| Exon 9 | G/A | 1312 | Va1405Ile | G=96.7% A=3.3% |
| Exon 9 | G/A | 1347 | None | G=96.7% A=3.3% |
| Exon 10 | G/C | 1561 | Gly488Arg | G=96.7% C=3.3% |
| Exon 14 | A/C | 2028 | Leu643Phe | A=90% C=10% |

OATP2 above refers to the clone sequenced by Hsiang et al (ref 1). Some comment on the numbering of exons in OATPC is required. This gene contains an exon (38 bp) upstream (5' UTR region) of the exon containing the ATG start site for translation. Therefore the exon numbering could vary depending whether this exon is counted as the first exon or not. In the literature, Konig (2000) JBC 275: 23161-68, have defined exon 1 as that containing the ATG start site and therefore we have adopted the same numbering in this application (but note that the priority document relating to the present application did vice versa; for example, exon 5 in this application is equivalent to exon 6 in the priority document).

| **PCR PRODUCTS** | | |
|---|---|---|
| **Fragment** | **Forward Oligo** | **Reverse Oligo** |
| 443-999 | 443-466 | 979-999 |
| 874-1360 | 874-896 | 1337-1360 |
| 1255-1684 | 1255-1278 | 1663-1684 |
| 1559-2095 | 1559-1581 | 2073-2095 |

| **RFLP analysis** | | | |
|---|---|---|---|
| **Polymorphism** | **Position** | **RFLP Enzyme/PCR size** | **RLFP fragment size** |
| G/A | 510 | | |
| C/T | 670 | BmR I/ 595bp | C=349bp, 246bp, T=595bp |
| C/T | 696 | | |
| C/G | 1299 | Apo I/ 595bp | C=30bp, 60bp, 380 bp G=90bp, 380bp |
| G/A | 1312 | Bst 4CI/ 595bp | G=77bp, 393bp A=470bp |
| G/A | 1347 | | |
| G/C | 1561 | HpyCH4IV/ 595bp | G=470bp C=144bp, 326bp |
| A/C | 2028 | Ase I/ 595bp | A=89bp,488bp C=577bp |

### Example 2

| **Further OATPC Polymorphisms** | | | | | | | |
|---|---|---|---|---|---|---|---|
| **SNPs in OATPC 3'UTR** (positions according to SEQ ID NO 1) | | | | | | | |
| | | | | | | | |

| **Exon** | **Nucleotide** | **SNP** | | | | **Frequency** | **Frequency** |
|---|---|---|---|---|---|---|---|
| | | | | | | **Caucasian** | **Japanese** |
| 3' UTR | 2327 | Ins T | | | | not screened | 0.1 |
| 3' UTR | 2342 | T>C | | | | not screened | 0.4 |
| | | | | | | | |
| | | | | | | | |

| **SNPs in OATPC promoter** (positions according to SEQ ID NO 3) | | | | | | | |
|---|---|---|---|---|---|---|---|
| | | | | | | | |

| | **Nucleotide** | **SNP** | | | | **Frequency** | **Frequency** |
|---|---|---|---|---|---|---|---|
| | | | | | | **Caucasian** | **Japanese** |
| | 321 | T>G | | | | 0.03 | not screened |
| | 1332 | A>C | | | | 0.08 | not screened |
| | | | | | | | |
| | | | | | | | |

| **SNPs in OATPC introns** | | | | | | | |
|---|---|---|---|---|---|---|---|
| | | | | | | | |

| **Intron** | **Nucleotide** | **SNP** | **Nucleotide** | **Sequence** | | | |
|---|---|---|---|---|---|---|---|
| | **position in** | | **position** | **ID No** | | | |
| | **relation to exon** | | **in sequence** | | | | |
| | | | | | | | |
| 1 | IVS1+21 | T>A | 41 | 4 | | | |
| 2 | IVS2+89 | T>G | 109 | 5 | | | |
| 2 | IVS2+224 | A>G | 244 | 5 | | | |
| 3 | IVS3+97 | C>A | 117 | 6 | | | |
| 3 | IVS3+263 | G>A | 283 | 6 | | | |
| 4 | IVS4+189 | G>A | 209 | 7 | | | |
| 4 | IVS4+191 | G>A | 211 | 7 | | | |
| 4 | IVS5-118 | deICTTGTA | 63 | 8 | | | |
| 6 | IVS6+33 | C>T | 53 | 9 | | | |
| 9 | IVS10-107 | ins TTC | 75 | 10 | | | |
| 11 | IVS11+142 | Ins T | 162 | 11 | | | |
| 12 | IVS13-97 | G>C | 84 | 12 | | | |

### OATPC intronic SNPs

### Key

20bp of exon sequence shown in uppercase
Intron sequence in lowercase (200 to 300bp only)
SNP shown in uppercase (one allele only)

### OATPC promoter region

Total length of the sequence = 1538bp
1500bp of OATPC sequence directly upstream from the cDNA sequence
Sequence in uppercase represents 38bp overlap with the cDNA sequence (SEQ ID NO 1) where this 38bp is 5'UTR sequence.
Nucleotide positions in the promoter have been determined where the -1 position is the base (lowercase) directly upstream of the end of the cDNA sequence.

## Claims

1. A method for the detection of a polymorphism in OATPC in a human, which method comprises determining the sequence of the human at:
position 1561 of the OATPC gene as defined by the position in SEQ ID NO: 1 is not G; or
position 488 in OATPC polypeptide defined by position in SEQ ID NO: 2 is not Gly.

2. Use of a method as defined in claim 1 to assess the pharmacogenetics of a drug transportable by OATPC.

3. A polynucleotide comprising at least 20 bases of the human OATPC gene and comprising the allelic variant:
| Region | variant | Position in SEQ ID NO | SEQ ID NO |
|---|---|---|---|
| Exon 10 | C | 1561 | 1 |

4. An ARMS allele specific primer or an allele-specific oligonucleotide probe selected from one of the following:
an ARMS allele specific primer; or
an allele-specific oligonucleotide probe,
capable of detecting a OATPC gene polymorphism as defined in claim 1.

5. Use of an OATPC polymorphism as defined in claim 1 as a genetic marker in a linkage study.

6. Use of a statin in preparation of a medicament for treating a cardiovascular disease in a human detected as having an OATPC polymorphism at one or more of the following positions:
position 1561 of the OATPC gene as defined by the position in SEQ ID NO: 1 is not G;
position 488 in OATPC polypeptide defined by position in SEQ ID NO: 2 is not Gly.

7. A use according to claim 6 in which the drug is rosuvastatin.

8. An allelic variant of human OATPC polypeptide comprising;
an arginine at position 488 of SEQ ID NO 2
or a fragment thereof comprising at least 10 amino acids provided that the fragment comprises the allelic variant.

## Patentansprüche

1. Verfahren zum Nachweis eines Polymorphismus in OATPC in einem Menschen, wobei man in dem Verfahren bestimmt, daß die Sequenz des Menschen in:
Position 1551 des OATPC-Gens, wie durch die Position in SEQ ID NO: 1 definiert, nicht G ist; oder
Position 488 im OATPC-Polypeptid, definiert durch die Positionen in SEQ ID NO: 2, nicht Gly ist.

2. Verwendung eines Verfahrens gemäß Anspruch 1 zur Beurteilung der Pharmacogenetik eines durch OATPC transportierbaren Arzneistoffs.

3. Polynukleotid, umfassend wenigstens 20 Basen des menschlichen OATPC-Gens und umfassend die Allelvariante:
| Region | Variante | Position in SEQ ID NO: | SEQ ID NO: |
|---|---|---|---|
| Exon 10 | C | 1561 | 1 |

4. ARMS-Allel-spezifischer Primer oder Allelspezifische Oligonukleotidsonde, ausgewählt aus einem der folgenden Moleküle:
einem ARMS-Allel-spezifischen Primer; oder
einer Allel-spezifischen Oligonukleotidsonde,
womit ein OATPC-Gen-Polymorphismus gemäß Anspruch 1 nachgewiesen werden kann.

5. Verwendung eines OATPC-Polymorphismus gemäß Anspruch 1 als genetischer Marker in einer Verknüpfungsuntersuchung.

6. Verwendung eines Statins bei der Herstellung eines Arzneimittels zur Behandlung einer Herzkreislauferkrankung in einem Menschen, bei dem ein OATPC-Polymorphismus an einer oder mehreren der folgenden Positionen nachgewiesen wurde:
Position 1561 des OATPC-Gens, wie durch die Positionen in SEQ ID NO: 1 definiert, ist nicht G;
Position 488 im OATPC-Polypeptid, definiert durch die Positionen in SEQ ID NO: 2, ist nicht Gly.

7. Verwendung nach Anspruch 6, wobei es sich bei dem Arzneistoff um Rosuvastatin handelt.

8. Allelvariante des menschlichen OATPC-Polypeptids, umfassend:
ein Arginin in Position 488 der SEQ ID NO: 2
oder ein Fragment davon, umfassend wenigstens 10 Aminosäuren, vorausgesetzt, daß das Fragment die Allelvariante umfaßt.

## Revendications

1. Procédé pour la détection d'un polymorphisme dans un OATPC d'un être humain, lequel procédé comprend l'étape consistant à déterminer la séquence de l'humain au niveau de :
la position 1561 du gène d'OATPC, comme cela est défini par la position dans la SEQ ID n° : 1, qui n'est pas un G ; ou
la position 488 dans un polypeptide d'OATPC, définie par la position dans la SEQ ID n° : 2, qui n'est pas une Gly.

2. Utilisation d'un procédé, comme cela est défini à la revendication 1, pour évaluer les aspects pharmacogénétiques d'une substance médicamenteuse pouvant être transportée par un OATPC.

3. Polynucléotide comprenant au moins 20 bases du gène d'OATPC humain et comprenant le variant allélique :
| Région | Variant | Position dans la SEQ ID n° : | SEQ ID n° : |
|---|---|---|---|
| Exon 10 | C | 1561 | 1 |

4. Amorce spécifique d'un allèle ARMS ou sonde oligonucléotidique spécifique d'un allèle choisie parmi l'un des éléments suivants :
une amorce spécifique d'un allèle ARMS ; ou
une sonde oligonucléotidique spécifique d'un allèle,
capable de détecter un polymorphisme dans un gène d'OATPC, comme cela est défini à la revendication 1.

5. Utilisation d'un polymorphisme d'OATPC, comme cela est défini à la revendication 1, en tant que marqueur génétique dans une étude de liaison.

6. Utilisation d'une statine dans la préparation d'un médicament destiné à traiter une affection cardiovasculaire chez un être humain, chez qui on a détecté un polymorphisme d'OATCP au niveau d'une ou plusieurs positions parmi les suivantes :
la position 1561 du gène d'OATPC, comme cela est défini par la position dans la SEQ ID n° : 1, n'est pas un G ; ou
la position 488 dans un polypeptide d'OATPC, définie par la position dans la SEQ ID n° : 2, n'est pas une Gly.

7. Utilisation selon la revendication 6, dans laquelle la substance médicamenteuse est la rosuvastatine.

8. Variant allélique d'un polypeptide d'OATPC humain comprenant :
une arginine à la position 488 de la SEQ ID n° : 2 ou un fragment de celle-ci comprenant au moins 10 acides aminés, à condition que le fragment comprenne le variant allélique.
